# EUROPEAN PATENT APPLICATION

(11) **EP 1 044 963 A2**
(43) Date of publication of application: **18.10.2000**
(21) Application number: 00105197.8
(22) Date of filing: 13.03.2000
(51) Int. Cl.: C07C 303/02, C07C 303/44, B01J 27/32, B01J 38/02

(54) **Process for the recovery of perfluorinated sulphonic acids from spent acid**

(30) Priority: 30.03.1999 US 126898 P
(71) Applicant: Haldor Topsoe A/S, DK-2800 Lyngby (DK)
(72) Inventor: Hommeltoft, Sven Ivar, 3400 Hillerod (DK)

(57) **Abstract**

Method for the recovery of a fluorinated sulphonic acid from a spent acid product by steps of
subjecting the spent acid product to thermal treatment to obtain the acid in its active form and a residue, and
separating the active acid from the residue by stripping with a stripping agent, comprising the further steps of
separating the striping agent from the active acid by distillation and withdrawing the separated stripping agent.

## Description

The present invention is directed to the recovery of fluorinated sulphonic acid from spent acid and in particular to a method, wherein the acid is recovered at high purity by means of a combined stripping and distillation process.

Fluorinated sulphonic acids are known as useful catalysts in a number of hydrocarbon conversion processes including alkylation of hydrocarbons. An example of those processes is alkylation of isobutane with lower olefins such as propene and butenes as described in US patent Nos. 5,220,095, 5,245,100 and 5,498,820. These processes typically produce various amounts of residue, in which the fluorinated sulphonic acid is in its inactive form by interaction with certain hydrocarbons in the residue. In order to ensure feasible economics, the acid catalyst has to be recovered from the residue and recycled to the process. Residue containing fluorinated sulphonic acid in its inactive form is referred to herein before and in the following description as spent acid.

Acid catalyst recovery from spent acid by treatment with sulphuric acid is disclosed in European patent No. 614,699. Recovery of acid catalyst by treatment with esters of sulphuric acid is known from DK patent No. 170,396. Furthermore, the acid may be extracted from spent acid with water and subsequent separation from the aqueous solution by either distillation of a salt of the acid with sulphuric acid as disclosed in DK patent No. 171,409 or by distillation after addition of a weak base in form of a salt of the acid to the aqueous solution to facilitate separation of the acid and water (US Patent Nos. 5,603,817 and 5,759,357). Acid recovery by hydrogenation of spent acid in presence of a hydrogenation catalyst is disclosed in US Patent No. 5,472,921.

In co-pending DK patent application no. PA 1998 00995, we describe a method, wherein fluorinated sulphonic acids are recovered from spent acid by thermal treatment of the substrate and stripping with a condensable stripping agent.

When the fluorinated sulphonic acid is stripped off from spent acid in the latter method, it will be an advantage to recover the stripping agent substantially free of the acid and to recycle the agent to the process.

It has been found that when carrying out recovery of fluorinated sulphonic acid at distillation conditions in a distillation column and employing a specific stripping agent, the agent will be recovered at top of the distillation column essentially free of acid after having passed a sufficient number of theoretical plates between the region from which the acid is withdrawn and the top of the column, where the stripping agent leaves the column.

Accordingly, this invention provides a method for the recovery of a fluorinated sulphonic acid from a spent acid product comprising steps of subjecting the spent acid product to thermal treatment to obtain the acid in its active form and a residue, and separating the active acid from the residue by stripping with a stripping agent, wherein the stripping agent is selected from a hydrocarbon being able to dissolute at least part of the active acid at the distillation conditions and the stripping agent is separated from the active acid by distillation.

During separation of the stripping agent and the active acid, the solubility of fluorinated sulphonic acid shall be higher in the liquid phase mixture of the stripping agent and the acid, than the content of the acid in the vapour phase of the mixture. In order to avoid azeotrope, it is therefore necessary that the striping agent has a high vapour pressure. Appropriate stripping agents for use in the above process are any hydrocarbons, which dissolute at least part of the active acid and do not form an azeotrope with the acid.

When employing the above process in recovery of trifluoromethanesulphonic acid from spent acid preferred hydrocarbons for use as stripping agent are those, which have a boiling point below 36°C at ambient pressure.

At present the most preferred stripping agent in the recovery of trifluoromethane sulphonic acid by the inventive process is n-pentane.

The above features and aspects of the invention will be illustrated in more detail in the following description by means of examples of specific embodiments of the invention.

### Example 1

### Stripping of spent acid with n-pentane at 250°C:

The equipment used for this experiment is illustrated in attached Figure.

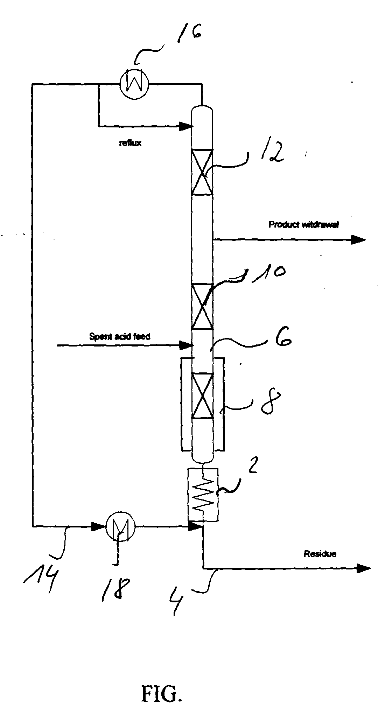

The lower part of the apparatus shown in the Figure consists of a glass spiral (ID=10 mm) 2 heated to about 252°C on the outside by condensing tetradecane. Distillation residue was withdrawn at bottom 4 of the glass spiral. On top of the spiral was placed a distillation column 6 (ID=3 cm, height=21 cm, packed with 4 mm Wilson helices) heated on the outside by condensing tetradecane to about 252°C. Spent acid was introduced between the heated column segment 8 and the heated glass spiral 2. Over the heated column segment was placed an insulated column segment 10 (height=18 cm) from top of which the active acid product was withdrawn. A third column segment 12 (28 cm) was arranged on the insulated segment from which the recovered stripping agent 14 was withdrawn and cooled in cooler 16, 18. The cooled stripping agent was then partly refluxed and recycled.

Spent acid from an isobutane alkylation process employing trifluoromethanesulphonic acid catalyst was used in this example. A stripping agent consisting of approximately 5 g/min n-pentane was evaporated and preheated before introduction into the glass spiral. About 15 g/min. of the condensed pentane were recycled to the top of the column as reflux. The rest of the pentane withdrawn from the top of the distillation column was recycled as stripping agent.

Over a period of 3 hrs 763 g of spent acid containing 64.4% trifluoromethanesulphonic acid were fed to the column. 660 g of a product were recovered consisting of a phase with 526 g of active acid at 90.4% purity and of a second phase of 134 g containing hydrocarbon and 1.4% trifluoromethanesulphonic acid. 125 g residue containing 24% trifluromethanesulphonic acid was withdrawn at bottom of the heated glass spiral. The content of trifluoromethanesulphonic acid in the recovered stripping agent was less than 0.1%.

### Comparison Example 2

### Stripping of spent acid with n-hexane at 250°C (reference example):

Example 2 was performed with the equipment as described in the above Example 1. The conditions used were similar to that of Example 1 with the exception that n-hexane was used as striping agent.

623 g of spent acid (64% trifluoromethanesulphonic acid) were to the column over a period of 2 hrs. 319 g product were withdrawn as two phases: 244 g active acid phase (89.6% acid) and 69 g hydrocarbon phase (0.4% acid). The residue recovered at bottom of the heated glass spiral proved contained 52% trifluoromethanesulphonic acid.

The condensed hexane-stripping agent was super-saturated with trifluoromethanesulphonic acid, which separated into an acid phase and hexane phase after condensation. 60 g of the acid phase with 93% trifluoromethanesulphonic acid were recovered from the 500 ml stripping agent reservoir at top of the column at the end of the test run.

The striping agent recycled to the column was thus saturated with acid. The higher acid content in the residue from this example compared to the acid content in the residue of Example 1 is a consequence of the stripping agent being saturated with acid.

## Claims

1. Method for the recovery of a fluorinated sulphonic acid from a spent acid product by steps of subjecting the spent acid product to thermal treatment to obtain the acid in its active form and a residue, and separating the active acid from the residue by stripping with a stripping agent, comprising the further steps of separating the striping agent from the active acid by distillation and withdrawing the separated stripping agent.

2. Process of claim 1, wherein the fluorinated sulphonic acid is a perfluoroalkane sulphonic acid.

3. Process of claim 1, wherein the fluorinated sulphonic acid is trifluoromethanesulphonic acid.

4. Process of claim 1, wherein the stripping agent is selected from hydrocarbons condensing at conditions during the separation step.

5. Process of claim 1, wherein the pressure is adjusted to obtain condensation of the stripping agent.

6. Process as in claim 1-5, wherein the stripping agent is recycled to the recovery process.

7. Process according to anyone of the preceding claims, wherein the stripping agent is selected from a hydrocarbon being able to dissolute at least part of the active acid at the distillation conditions and the stripping agent is separated from the active acid by distillation
